Europäisches Patentamt

European Patent Office

Office européen des brevets

(19)

(11) Veröffentlichungsnummer: **0 006 158**
**B1**

(12) **EUROPÄISCHE PATENTSCHRIFT**

(45) Veröffentlichungstag der Patentschrift: **03.02.82**

(21) Anmeldenummer: **79101674.4**

(22) Anmeldetag: **31.05.79**

(51) Int. Cl.³: **G 01 N 33/48, A 61 L 15/06**

(54) Testpflasterstreifen.

(30) Priorität: **09.06.78 DE 7817327 U**

(43) Veröffentlichungstag der Anmeldung:
**09.01.80 Patentblatt 80/1**

(45) Bekanntmachung des Hinweises auf die Patenterteilung:
**03.02.82 Patentblatt 82/5**

(84) Benannte Vertragsstaaten:
**AT BE CH DE FR GB IT NL SE**

(56) Entgegenhaltungen:
**DE - C - 1 598 463
DE - U - 1 685 261
DE - U - 7 519 341
DE - U1 1 755 526
US - A - 3 212 495**

(73) Patentinhaber: **Maucher, Ortrun Mara, Dr.
Richard-Wagner-Strasse 15
D-7800 Freiburg i.Br. (DE)**

(84) **CH DE FR GB IT NL SE AT**

(73) Patentinhaber: **Maucher, Wolfgang
Richard-Wagner-Strasse 15
D-7800 Freiburg i-Br. (DE)**

(84) **BE CH**

(72) Erfinder: **Maucher, Ortrun Mara, Dr.
Richard-Wagner-Strasse 15
D-7800 Freiburg i.Br. (DE)**

(74) Vertreter: **Patentanwälte Dipl.-Ing. Hans Schmitt
Dipl.-Ing. Wolfgang Maucher
Dreikönigstrasse 13
D-7800 Freiburg i.Br. (DE)**

Courier Press, Leamington Spa, England.

Testpflasterstreifen

Die Erfindung betrifft einen Testpflasterstreifen zum gleichzeitigen Aufbringen von mehreren Allergenen vorzugsweise unter Luftabschluß, mit mehreren saugfähigen Bereichen, diese umgrenzenden und teilweise übergreifenden Randabdeckungen und wenigstens einer Klebefläche, wobei die Randabdeckungen als die Reizwirkung der Klebemasse der Klebeflächen in unmittelbarer Umgebung der als Teststellen dienenden saugfähigen Bereiche ausschaltende Schutzzone dienen.

Ein derartiger Testpflasterstreifen ist aus US—A—3 212 495 vekannt. Dabei ist für jeden saugfähigen Bereich eine eigene Randabdeckung vorgesehen und zwischen den ein-zelnen Randabdeckungen sind weitere Klebe-flächen. Die einzelnen Testbereiche sind also jeweils von Klebeflächen umschlossen. Durch diese großen Klebeflächen ist die Handhabung dieses bekannten Testpflasters erschwert, weil vor allem die Gefahr besteht, daß diese großen Klebeflächen an den Fingern des Benutzers hängenbleiben. Darüber hinaus läßt sich trotz relativ großer Klebeflächen mit diesen ein solches Testpflaster nicht genügend sicher über eine genügend lange Zeit fixieren, so daß häufig zusätzliche Haltepflaster benötigt werden. Dann ist aber die relativ große Klebefläche nicht sinnvoll.

Es besteht deshalb die Aufgabe, einen Testpflasterstreifen der eingangs erwähnten Art zu schaffen, bei welchem die Handhabung dadurch verbessert ist, daß der Benutzer genügend Möglichkeiten zum Erfassen des Testpflasterstreifens hat, ohne daß der Testpflasterstreifen an seinen Fingern haftern bleibt. Zusätzlich soll die Abgrenzung zwischen Hautreaktionen im Testbereich und durch die Befestigung hervorgerufene Reaktionen verbessert sein.

Die Lösurig dieser Aufgabe besteht darin, daß wenigstens ein mehrere Lochungen aufweisender Streifen, dessen Oberfläche inert ist, die Randabdeckungen bildet und die saugfähigen Bereiche hinter den Lochungen des Lochstreifens angeordnet sind.

Dadurch wird ein Testpflasterstreifen zur Verfügung gestellt, bei welchem nach dem Aufbringen der Allergene sogleich eine Fixierung an der Haut möglich ist. Die Klebeflächen sind dabei durch den Lochstreifen so relativ schmal, daß ein Festkleben an den Fingern des Benutzers praktisch ausgeschlossen oder zumindest in seiner Wirkung stark reduziert ist. Gleichzeitig ergeben sich Shutzzonen, die eventuelle Reaktionen auf den Klebstoff von Testreaktionen scharf trennen. Gegenüber dem Testpflasterstreifen nach der US—A—3 212 495 ergibt sich auch der Vorteil, daß zwischen den Testbereichen keine durch Klebstoff hervorgerufenen Reaktionen auftreten können, weil dort der allergologisch inerte Lochstreifen durchgehend angeordnet ist. Dabei bleibt auch in wünschenswerter Weise der Vorteil erhalten, daß die saugfähigen Bereiche an ihren Rändern etwas übergriffen sind, so daß ein verdeckter Saugbereich zur Aufnahme überschüssigen Mediums geschaffen ist. Dabei ist die Herstellung einfach, da zur Schaffung dieser Randabdeckung und der Schutzzone ein Loch-streifen auf das Pflastergrundgewebe auf-gebracht sein kann, wobei dieser Lochstreifen eine Doppelfunction erhält.

Eine Ausgestaltung des Testpflasterstreifens kann darin bestehen, daß der Lochstreifen wenigstens einen Lângsrandbereich, vorzugs-weise zwei einander gegenüberliegende Längs-ränder des Pflasters als Klebeflächen frei läßt. Dabei ist es besonders vorteilhaft, wenn die Schutzzonen eine rechteckige Außenkontur haben und die Testbereiche vorzugsweise kreisrund sind. Falls sowohl eine Testreaktion als auch eine Reaktion auf die Klebestreifen auftritt, können diese ohne weiteres gut vonein-ander unterschieden werden, zumal sich dann ein deutlicher, der Geometrie des erfindungsgemäßen Testpflasterstreifens entsprechender Reaktionsbereich auf der Haut bildet.

Dabei kann eine abgewandelte Ausführungsform der Erfindung darin bestehen, daß der Lochstreifen selbst mit einem eine Schutzzone zu seinen Lochungen bildenden Abstand Klebeflächen oder Klebestreifen aufweist.

Besonders vorteilhaft ist es, wenn der Testpflasterstreifen mit fünf in zumindest einer Reihe nebeneinander angeordneten saugfähigen Bereichen ausgebildet ist. Die Verwendung von fünf Testbereichen an einem derartigen Streifen kommt vor allem dem Dezimalsystem entgegen, erlaubt eine gleichzeitige Aufbringung einer relativ großen Anzahl von Allergenen, ohne dabei einen zu großen Streifen zu bilden, der dann wieder schwerer zu handhaben wäre.

Gegebenenfalls können auch mehrere parallele Reihen von saugfähigen Bereichen vorgesehen sein, die entweder von einem gemeinsamen oder von mehreren jeweils parallel zueinander angeordneten Lochstreifen abgedeckt sein können.

Weitere Ausgestaltungen der Erfindung sind in weiteren Ansprüchen enthalten.

Nachstehend ist die Erfindung mit ihren ihr als wesentlich zugehörenden Einzelheiten anhand der Zeichnung noch näher beschrieben. Es zeigt:

Fig. 1 eine Draufsicht eines erfindungsgemäßen Testpflasterstreifens mit fünf Testbereichen nach dem Entfernen einer Abdeckfolie,

Fig. 2 in vergrößertem Maßstab einen Querschnitt durch einen Testbereich,

Fig. 3 in schaubildlicher Darstellung einen Bereich eines Testpflasterstreifens, bei welchem durch eine Unterbrechung des Abdeck-Lochstreifens ein quer verlaufender Klebestreifen gebildet ist, sowie

Fig. 4 einen Querschnitt durch den Testbereich einer abgewandelten Ausführungsform eines erfindungsgemäßen Testpflasterstreifens.

Ein im ganzen mit 1 bezeichneter Testpflasterstreifen dient in bekannter Weise zum Aufbringen von Allergenen. Dabei werden die Allergene jeweils von einem saugfähigen Bereich 2 aufgenommen, welcher von einer ihn teilweise übergreifenden Randabdeckung umgrenzt ist. Mittels Klebestreifen 4 kann dieser Testpflasterstreifen 1 vorläufig oder bei genügend starker Ausbildung dieser Klebestreifen 4 auch endgültig fixiert werden. Eine endgültige Befestigung kann außerdem in nicht näher dargestellter Weise mit zusätzlichen Pflastern erfolgen, die nachträglich über die Rückseite des Testpflasterstreifens 1 und die Haut des Patienten gelegt werden. Dadurch wird sichergestellt, daß dieser Testpflasterstreifen auch während seines Aufbringens und Fixierens praktisch unverschiebbar ist, so daß die saugfähigen Bereiche als genau definierte Testbereiche auf die Haut eines Patienten einwirken können.

Erfindungsgemäß ist vorgesehen, daß als Randabdeckung ein Lochstreifen 8 dient, hinter dessen Lochungen 3 die saugfähigen Bereiche 2 angeordnet sind. Die Oberfläche des Lochstreifens 8 ist dabei allergologisch inert, so daß der Lochstreifen 8 als die Reizwirkung der Klebemasse der Klebeflächen oder Klebestreifen 4 in unmittelbarer Umgebung der als Teststellen dienenden saugfähigen Bereiche 2 ausschaltende Schutzzone 5 dient. Dadurch ist einerseits eine gute Handhabung des Testpflasterstreifens 1 möglich, da ein Benutzer gut an diesen Bereichen 5 mit den Fingern anfassen kann, ohne daß das Pflaster 1 an ihm haften bleibt. Gleichzeitig sind die Klebeflächen oder -streifen 4 in ihrer Größe so stark reduziert, daß auch im Falle einer Pflasterreizung die eigentliche Testablesung auf der Haut des Patienten nicht gefährdet oder erschwert wird. Dennoch sind diese Klebestreifen 4 groß genug, um das Pflaster 1 zumindest vorläufig ausreichend fest fixieren zu können, bis es endgültig befestigt wird. Die dabei gebildete Schutzzone 5 hat im Ausführungsbeispiel eine rechteckige Außenkontur, während die Testbereiche 2 kreisrund sind. Diese geometrischen Verhältnisse tragen zu einer weiteren Verbesserung der Unterscheidbarkeit bei gleichzeitiger Reizung durch die Klebemasse und das Allergen bei.

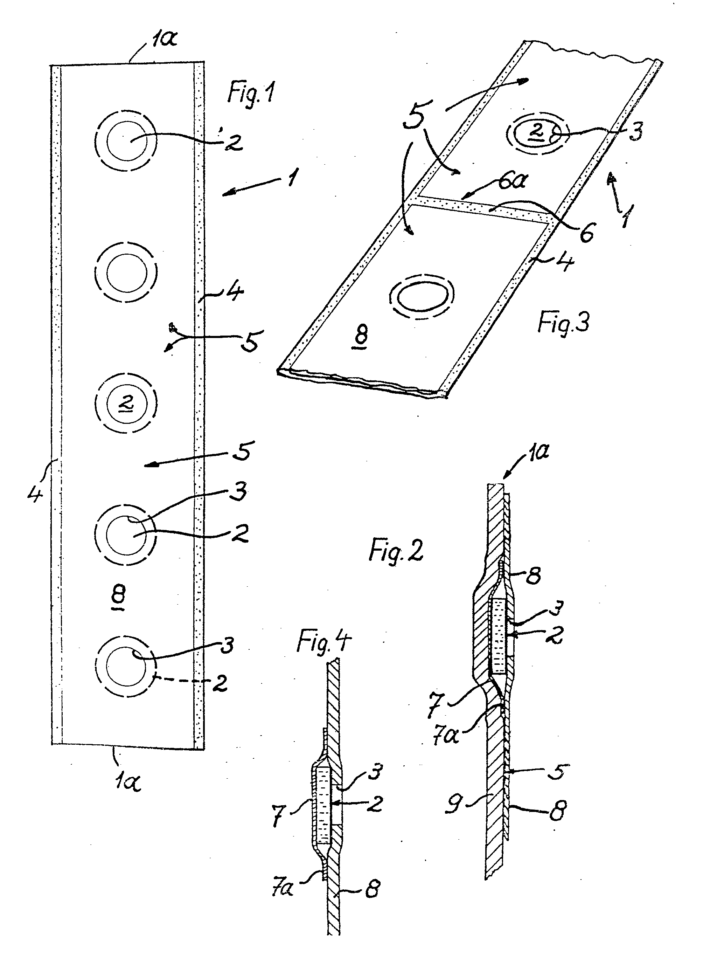

Der Lochstreifen 8 läßt wenigstens einen Längsrandbereich, im Ausführungsbeispiel zwei einander gegenüberliegende Längsränder des Pflastergrundgewebes 9 als Klebeflächen 4 frei, was man deutlich in Fig. 1 erkennt. Er ist im Ausführungsbeispiel mit fünf in zumindest einer Reihe nebeneinander angeordneten saugfähigen Bereichen 2 ausgebildet und die erwähnten Klebestreifen 4 laufen entlang den längeren Längsrändern diese Streifens. Trotz des relativ langen Testpflasterstreifens ist durch diese sinnreiche Anordnung der Klebeflächen und des klebstoffreien Lochstreifens einerseits ein vorläufiges Fixieren möglich, andererseits jedoch ein Verkleben mit der Hand der testenden Person im wesentlichen vermieden. Dadurch wird die Handhabung vereinfacht und sicherer, da bei einem kurzzeitigen Verkleben mit der Hand eines Benutzers unter Umständen schon aufgebrachte Allergene benachbarter Bereiche miteinander in Berührung kommen könnten. Selbst ein Auftragen von etwas zu viel Allergen auf den einen oder anderen Testbereich 2 wird dabei durch die zusätzliche Funktion des Lochstreifens 8 auch als Randabdeckung der saugfähigen Bereiche 2 unschädlich gemacht.

Die Oberfläche des Lochstreifens 8 kann zum Beispiel mit Aluminium beschichtet, insbesondere bedampft oder mit Aluminiumfolie od.dgl. abgedeckt sein. Darüberhinaus kann der Lochstreifen 8 selbst aus Aluminiumfolie bestehen. Dadurch wird dieser Lochstreifen auf einfache Weise allergologisch im wesentlichen inert, so daß in der von dem Lochstreifen gebildeten Schutzzone 5 Hautreaktionen in erwünschter Weise ausgeschlossen sind.

Will main eine noch bessere Fixierung des erfindungsgemäßen Testpflasterstreifens 1 erreichen, können gemäß Fig. 3 auch mehrere Lochstreifen 8 mit Unterbrechungen 6a zwischen den Lockstreifen vorgesehen sein, und diese Unterbrechungen 6a können als quer verlaufende Klebstreifen 6 ausgebildet sein. Insbesondere auch noch längere Testpflasterstreifen mit mehr als fünf Testbereichen könnten auf diese Weise gebildet werden. So wäre zum Beispiel ein Testpflasterstreifen mit zehn derartigen Bereichen möglich, wobei die Lochstreifen 8 aber die gleiche Länge wie die von Testpflasterstreifen mit fünf Testbereichen haben könnten. Durch solche quer verlaufende Klebstreifen 6 kann die vorläufige oder auch die endgültige Fixierung des Streifens 1 verbessert werden. Darüberhinaus ist es dadurch möglich, den Pflasterstreifen 1 zwischen den einzelnen Testbereichen zu zerchneiden und doch einen zusätzlichen schmalen Rand-Klebstreifen zu erhalten.

Die saugfähigen Bereiche 2, also die Testflächen, sind im Ausführungsbeispiel in der Mitte des Pflasterstreifens 1 nebeneinander angeordnet, halten zwischen sich jeweils etwa den gleichen Abstand und sind, wie schon erwähnt kreisförmig ausgebildet. Wenigstens eines der schmalen Enden 1a des Pflasterstreifens 1, im Ausführungsbeispiel nach Fig. 1 beide schmalen Enden 1a, sind klebstoffrei. Es ist nämlich erwünscht, gerade die Enden 1a des

5                    **0 006 158**                    6

Testpflasterstreifens 1 anfassen zu können. Darüberhinaus wird auf diese Weise die Möglichkeit einer Reizung der Haut durch Klebemasse weiter verringert.

Als saugfähiger Bereich kann in an sich bekannter Weise ein Baumwoll-Läppchen od.dgl. vorgesehen sein, dessen mit dem Allergen zu beschichtende Oberseite vorzugsweise aufgerauht ist. Dies ergibt eine große Saugfähigkeit. Unterhalb dieses den saugfähigen Bereich 2 bildenden Baumwoll-Läppchens kann gemäß Fig. 2 eine luftdichte Folie 7 angeordnet sein, wodurch die Allergene unter Lufabschluß auf die Haut einwirken können. Diese Folie 7 liegt dabei mit ihren Rändern 7a an der Unterseite des Lochstreifens 8 dicht an und ist vorzugsweise dort angeklebt oder verschweißt. Die Darstellung in Fig. 2 und der noch zu beschreibenden Fig. 4 ist dabei zur Verdeutlichung insbesondere hinsichtlich der Dickenabmessungen etwas übertrieben.

Es sei erwähnt, daß die Klebestreifen 4 und gegebenenfalls 6 vor der Benutzung mit einer insbesondere auch die saugfähigen Bereiche 2 abdeckenden Abziehfolie versehen sein können, wodurch die Testbereiche gut geschützt sind. Es sei erwähnt, daß zwischen wenigstens zwei einander benachbarten saugfähigen Bereichen 2 auch auf dem Lochstreifen 8 verlaufende Klebstreifen 6 vorgesehen sein könnten, um die Fixierbarkeit des Testpflasterstreifens 1 zu verbessern.

Schließlich könnten auch mehrere parallele Reihen von saugfähigen Bereichen 2 vorgesehen sein, die von einem gemeinsamen oder jeweils parallel zueinander angeordneten Lochstreifen 8 im Randbereich abgedeckt sind.

In Fig. 4 ist eine abgewandelte Ausführungsform der Erfindung angedeutet, bei welcher nämlich auf ein zusätzliches Pflastergewebe 9 verzichtet ist.

In diesem Falle weist der Lochstreifen 8 hinter seinen Lochungen 3 einzelne, die Teststellen bildende Baumwoll-Läppchen od.dgl. saugfähigen Bereiche 2 auf und hat außerdem selbst mit einem eine Schutzzone 5 zu seinen Lochungen 3 bildenden Abstand Klebeflächen oder Klebestreifen, was bei einer Draufsicht etwa das gleiche Bild wie Fig. 1 oder 3 ergäbe.

Insgesamt ergibt sich ein Testpflasterstreifen 1, welcher sich gut für eine Massenfertigung eignet und außerdem eine rationelle und schnelle Testung erlaubt, da einerseits die Handhabung und andererseits die Ablesegenauigkeit nach dem Abziehen des Streifens 1 verbessert sind und bei welchem auch das Aufbringen von etwas zu viel Allergen nicht zu einem Verschmieren und zu einem Unwirksamwerden der Schutzzone 5 führt. Dadurch eignet sich dieser Testpflasterstreifen besonders gut für eine Anwendung dort, wo zahlreiche Patienten in möglichst kurzer Zeit getestet werden müssen und für das Auflegen der Pflaster eventuell nur Hilfskräfte zur Verfügung stehen.

Alle in der Beschreibung, den Ansprüchen und der Zeichnung dargestellten Merkmale und Konstruktionsdetails können sowohl einzeln als auch beliebiger Kombination miteinander wesentliche Bedeutung haben.

**Patentansprüche**

1. Testpflasterstreifen zum gleichzeitigen Aufbringen von mehreren Allergenen vorzugsweise unter Luftabschluß, mit mehreren saugfähigen Bereichen, diese umgrenzenden und teilwiese übergreifenden Randabdeckungen und wenigstens einer Klebefläche, wobei die Randabdeckungen als die Reiswirkung der Klebemasse der Klebeflächen in unmittelbarer Umgebung der als Teststellen dienenden saugfähigen Bereiche ausschaltende Schutzzone dienen, dadurch gekennzeichnet, daß wenigstens ein mehrere Lochungen (3) aufweisender Streifen (8), dessen Oberfläche inert ist, die Randabdeckungen bildet und die saugfähigen Bereiche (2) hinter den Lochungen (3) des Lochstreifens (8) angeordnet sind.

2. Testpflasterstreifen nach Anspruch 1, dadurch gekennzeichnet, daß der Lochstreifen (8) wenigstens einen Längsrandbereich, vorzugsweise zwei einander gegenüberliegende Längsränder des Pflasters als Klebeflächen (4) frei läßt.

3. Testpflasterstreifen nach Anspruch 1 oder 2, dadurch gekennzeichnet, daß der Lochstreifen (8) selbst mit einem eine Schutzzone (5) zu seinen Lochungen (3) bildenden Abstand Klebeflächen oder Klebestreifen aufweist.

4. Testpflasterstreifen nach Anspruch 1 bis 3, dadurch gekennzeichnet, daß er als saugfähige Bereiche (2) Baumwoll-Läppchen aufweist.

5. Testpflasterstreifen nach einem der Ansprüche 1 bis 4, dadurch gekennzeichnet, daß die Schutzzonen (5) eine rechteckige Außenkontur haben und die Testbereiche (2) vorzugsweise kreisrund sind.

6. Testpflasterstreifen nach einem der Ansprüche 1 bis 5, dadurch gekennzeichnet, daß die Oberfläche des Lochstreifens (8) mit Aluminium beschichtet, insbesondere bedampft oder mit Aluminiumfolie abgedeckt ist oder selbst aus Aluminiumfolie besteht.

7. Testpflasterstreifen nach einem oder mehreren der Ansprüche 1 bis 6, dadurch gekennzeichnet, daß mehrere Lochstreifen (8) durch Unterbrechungen (6a) getrennt sind und daß diese Unterbrechungen (6a) vorzugsweise als quer verlaufende Klebestreifen (6) ausgebildet sind.

8. Testpflasterstreifen nach einem oder mehreren der Ansprüche 1 bis 7, dadurch gekennzeichnet, daß er mit fünf zn zumindest einer Reihe nebeneinander angeordneten saugfähigen Bereichen (2) ausgebildet ist.

9. Testpflasterstreifen nach einem oder mehreren der Ansprüche 1 bis 8, dadurch gekennzeichnet, daß die saugfähigen Bereiche (2) in der Mitte des Pflasterstreifens (1) nebeneinander angeordnet sind, zwischen sich jeweils etwa den gleichen Abstand halten und vorzugsweise kreisförmig ausgebildet sind.

10. Testpflasterstreifen nach einem oder mehreren der Ansprüche 1 bis 9, dadurch gekennzeichnet, daß wenigstens eines der schmalen Enden (1a) des Pflasterstreifens klebstofffrei ist.

11. Testpflasterstreifen nach einem oder mehreren der Ansprüche 1 bis 10, dadurch gekennzeichnet, daß unterhalb des saugfähigen Bereiches (2) eine luftdichte Folie (7) angeordnet ist, die mit ihren Rändern (7a) vorzugsweise an der Unterseite der Randabdeckung (3) dicht anliegt, gegebenenfalls angeklebt oder verschweißt ist.

12. Testpflasterstreifen nach einem oder mehreren der Ansprüche 1 bis 11, dadurch gekennzeichnet, daß mehrere parallele Reihen von saugfähigen Bereichen (2) vorgesehen sind, die entweder von einem gemeinsamen oder von mehreren jeweils parallel zueinander angeordneten Lochstreifen (8) abgedeckt sind.

## Claims

1. Sticking plaster test strip for simultaneously applying a plurality of allergens, preferably whilst excluding air, having a number of absorbent areas, edge coverings defining these absorbent areas and partially overlapping therewith, and at least one adhesive surface, the edge coverings serving as a protective zone which obviates the irritant effect of the adhesive composition of the adhesive surfaces in the immediate vicinity of the absorbent areas acting as test sites, characterised in that at least one strip (8) provided with a pluraltiy of perforations (3) and having an inert surface forms the edge coverings, and the absorbent areas (2) are arranged behind the perforations (3) in the perforated strip (8).

2. Sticking plaster test strip as claimed in claim 1, characterised in that the perforated strip (8) leaves at least one longitudinal edge portion, preferably two opposing longitudinal edges of the plaster exposed to act as adhesive surfaces (4).

3. Sticking plaster test strip as claimed in claim 1 or 2, characterised in that the perforated strip (8) itselt comprises adhesive surfaces or adhesive strips at a spacing which forms a protective zone (5) relative to its perforations (3).

4. Sticking plaster test strip as claimed in claims 1 to 3, characterised in that it has small cotton pads as the absorbent areas (2).

5. Sticking plaster test strip as claimed in one of claims 1 to 4, characterised in that the protective zones (5) have a rectangular outline and the test sites (2) are preferably circular.

6. Sticking plaster test strip as claimed in one of claims 1 to 5, characterised in that the surface of the perforated strip (8) is coated with aluminium, the aluminium coating being vapour-deposited, in particular, or is covered with aluminium foil or itself consists of aluminium foil.

7. Stricking plaster test strip as claimed in one or more of claims 1 to 6, characterised in that a plurality of perforated strips (8) are separated by interruptions (6a) and these interruptions (6a) are preferably in the form of transversely extending adhesive strips (6).

8. Sticking plaster test strip as claimed in one or more of claims 1 to 7, characterised in that it is formed with five adsorbent areas (2) arranged side by side in at least one row.

9. Sticking plaster test strip as claimed in one or more of claims 1 to 8, characterised in that the absorbent areas (2) are arranged side by side in the centre of the plaster strip (1), these areas (2) being at substantially the same spacing from each other and preferably being circular in form.

10. Sticking plaster test strip as claimed in one or more of claims 1 to 9, characterised in that at least one of the narrow ends (1a) of the plaster strip is free from adhesive.

11. Sticking plaster test strip as claimed in one or more of claims 1 to 10, characterised in that, beneath the absorbent area (2), there is an airtight film (7) which abuts closely with its edges (7a), preferably on the underside of the edge covering (3), and may be adhesively bonded or welded thereto.

12. Sticking plaster test strip as claimed in one or more of claims 1 to 11, characterised in that a number of parallel rows of absorbent areas (2) are provided, which are covered either by a common perforated strip (8) or by a plurality of perforated strips (8) arranged parallel to one another.

## Revendications

1. Bande d'emplâtre d'essai pour l'application simultanée de plusieures allergènes, de préférence à l'abri de l'air, comportant plusieurs régions absorbantes, des recouvrements de bord, entourant ces dernières et empiètant partiellement sur elles, et au moins une surface adhésive, les recouvrements de bord servant de zone protectrice qui supprime l'action irritante de l'adhésif des surfaces adhésives au voisinage immédiat des régions absorbantes, qui servent de zones d'essai, caractérisée en ce qu'au moins une bande (8) présentant plusieurs perforations (3), et dont la surface est inerte, forme les recouvrements de bord, et les régions absorbantes (2) sont disposées derrière les perforations (3) de la bande perforée (8).

2. Bande d'emplâtre d'essai selon la revendication 1, caractérisée en ce que la bande perforée (8) laisse libre, comme surfaces adhésives (4), au moins une région de bord longitudinal, de préférence deux bords longitudinaux opposés de l'emplâtre.

3. Bande d'emplâtre d'essai selon l'une des revendications 1 ou 2, caractérisée en ce que la bande perforée (8) présente elle-même des surfaces adhésives ou bandes adhésives qui présentent, relativement à ses perforations (3), un espacement formant une zone protectrice (5).

4. Band d'emplâtre d'essai selon les revendications 1 à 3, caractérisé en ce que comme régions absorbantes (2), elle présente des pattes de coton.

5. Bande d'emplâtre d'essai selon l'une des revendications 1 à 4, caractérisée en ce que les zones protectrices (5) présentent un contour rectangulaire (5) et les régions d'essai (2) sont de préférence circulaires.

6. Bande d'emplâtre d'essai selon l'une des revendications 1 à 5, caractérisée en ce que la surface de la bande perforée (8) est revêtue d'aluminium, en particulier vaporisée ou recouverte d'une feuille d'aluminium ou est elle même formée de feuille d'aluminium.

7. Bande d'emplâtre d'essai selon une ou plusieurs des revendications 1 à 6, caractérisée en ce que plusieurs bandes perforées (8) sont séparées par des interruptions (6a) et en ce que ces interruptions (6a) sont de préférence conçues sous la forme de bandes adhésives dirigées transversalement (6).

8. Bande d'emplâtre d'essai selon une ou plusieurs des revendications 1 à 7, caractérisée en ce qu'elle est munie de cinq régions absorbantes (2) disposées côte à côte en au moins une rangée.

9. Bande d'emplâtre d'essai selon une ou plusieurs des revendications 1 à 8, caractérisée en ce que les regions absorbantes (2) sont disposées côte à côte au milieu de la bande d'emplâtre (1), et en ce qu'elles ont chaque fois à peu près le même espacement entre elles, et elles sont de préférence de forme circulaire.

10. Bande d'emplâtre d'essai selon une ou plusieurs des revendications 1 à 9, caractérisé en ce qu'au moins une des extrémités étroites (1a) de la bande d'emplâtre est dépourvue d'adhésif.

11. Bande d'emplâtre d'essai selon une ou plusieurs des revendications 1 à 10, caractérisée en ce qu'en dessous de la région absorbante (2) est disposée une feuille étanche à l'air (7) qui, par ses bords (7a), s'applique de préférence tout contre la face inférieure du recouvrement de bord (3), et est éventuellement collée ou soudée.

12. Bande d'emplâtre d'essai selon une ou plusieurs des revendications 1 à 11, caractérisée en ce qu'elle comporte plusieurs rangées parallèles de régions absorbantes (2), qui sont recouvertes ou bien par une bande perforée commune (8) ou bien par plusieurs bandes perforées (8) chaque fois disposées parallèle-ment entre elles.

Fig.1

Fig.3

Fig.2

Fig.4